(19) Europäisches Patentamt
European Patent Office
Office européen des brevets

(11) Publication number: **0 019 893**
**B1**

(12) # EUROPEAN PATENT SPECIFICATION

(45) Date of publication of patent specification: **11.05.83**

(21) Application number: **80102918.2**

(22) Date of filing: **24.05.80**

(51) Int. Cl.³: **C 07 C 87/28,**
C 07 C 87/34, C 07 C 85/12
//C07C121/50, C07C119/042,
C08G18/74, C09D3/72

(54) **Novel triamines and method for production thereof.**

(30) Priority: **29.05.79 JP 67169/79**
**29.05.79 JP 67170/79**

(43) Date of publication of application:
**10.12.80 Bulletin 80/25**

(45) Publication of the grant of the patent:
**11.05.83 Bulletin 83/19**

(84) Designated Contracting States:
**AT BE CH DE FR GB IT LI NL SE**

(56) References cited:
**DE - A - 1 919 778**
**DE - A - 2 614 244**
**GB - A - 1 149 251**

(73) Proprietor: **Takeda Chemical Industries, Ltd.**
**27, Doshomachi 2-chome**
**Higashi-ku Osaka, 541 (JP)**

(72) Inventor: **Minato, Ichiro**
**4-14, Tsurukabuto 1-chome**
**Nada-ku Kobe, Hyogo 657 (JP)**
Inventor: **Furuoya, Itsuo**
**604, 33 Yamadanishi 3-chome**
**Suita, Osaka 565 (JP)**
Inventor: **Shibata, Koichi**
**55-12, Daiwahigashi 1-chome**
**Kawanishi, Hyogo 666-01 (JP)**

(74) Representative: **von Kreisler, Alek, Dipl.-Chem. et al,**
**Deichmannhaus am Hauptbahnhof**
**D-5000 Köln 1 (DE)**

**EP 0 019 893 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

Novel triamines and method for production thereof.

The present invention relates to novel triamines useful as raw materials for producing plastics, particularly polyurethane resins, and to processes for preparing the same.

Polyamine compounds such as tolylenediamine (TDA) and diaminodiphenylmethane as well as corresponding polyisocyanate compounds derived therefrom such as tolylene diisocyanate (TDI) and diphenylmethane diisocyanate (MDI) find their application not only as raw materials for polyurethane resins but also in many other areas, and have been used as the very important material. However, polyurethane compounds which are produced from these polyisocyanate compounds suffer from the great defect of inferior weatherability, or the tendency to yellow with time elapsed, and such defect constitutes one of the limitations on the application utility of these polyisocyanates.

As the result of a variety of efforts made so far to obtain the polyurethane compounds with improved weatherability, there have been already produced on a commercial scale polyamine compounds, such as hexamethylenediamine, xylylenediamine, hydrogenated xylylenediamine and isophoron diamine, and also polyisocyanate compounds derived from these polyamine compounds, such as hexamethylene diisocyanate (HDI), xylylene diisocyanate (XDI), hydrogenated xylylene diisocyanate ($H_6XDI$), and isophoron diisocyanate (IPDI), while different approaches are under way with the aim of their application to polyurethane resins. Nevertheless, these have a smaller number of functional groups per molecule and a high vapor pressure at ambient temperature, and are therefore required to be modified into adducts with polyfunctional alcohols or among isocyanate compounds themselves. Yet, the reduced available-isocyanate content of these adducts, being coupled with their raised viscosity, makes it quite difficult to formulate them into the solvent-free or high-solids coatings which currently are strongly demanded from the standpoint of strengthened control of the environmental pollution. In manufacturing such adducts, furthermore, complex manufacturing steps and costly facilities become required, so as to decrease their monomer content, which presents the hygienically serious problem at working sites where these resins are used.

As may be obvious from the above, strongly demanded are intermediate materials for the production of the polyisocyanates that are free from the defects of currently utilized raw materials and that, in the case of their application to polyurethane resins, can offer the superior weatherability and permit the manufacture of solvent-free or high-solids coatings.

The present inventors, with the specific view to producing the intermediate material provided with such requirements from a commercially available, relatively cheap starting material in simplified and lessened production steps, conducted extensive and comprehensive research study and came to complete the present invention.

Thus, the present invention relates to novel triamines represented by the following formula:

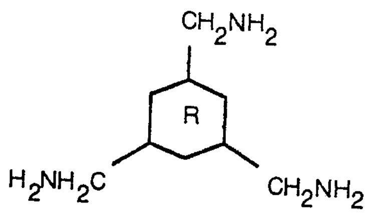 [I]

wherein 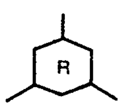 is 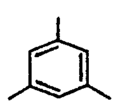 or 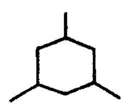

and to processes for producing the novel triamines [I].

When 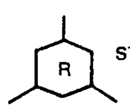 stands for 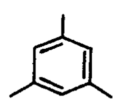

in the above formula [I], the triamine is 1,3,5-tris(aminomethyl)benzene (hereinafter referred to as "MTA"), and when

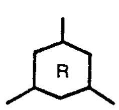 stands for 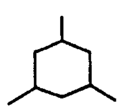

in the above formula [I], the triamine is 1,3,5-tris(aminomethyl)cyclohexane (hereinafter referred to as "$H_6$MTA"). That is, the triamines [I] include MTA and $H_6$MTA.

MTA can be produced by hydrogenation of 1,3,5-tricyanobenzene (hereinafter referred to as "MTN") in the presence of a catalyst.

$H_6$MTA can be produced by either hydrogenation of MTA in the presence of a catalyst, or direct hydrogenation of MTN in the presence of a catalyst.

The trinitrile MTN can be obtained by the ammoxidation of mesitylene existing abundantly in a distillate of petroleum. For example, a catalyst containing oxides and the like of vanadium, chromium, uranium, barium, germanium, hafnium, rhenium and thorium is filled in a conventional fixed-bed reactor, and a mixed gas composed of 0.1 to 3 mole % of mesitylene, 0.3 to 20 mole % of ammonia and 80 to 99 mole % of air is subjected to ammoxidation at atmospheric pressure at a space velocity of 300 to 3000 $hr^{-1}$, with the reaction temperature maintained at about 300 to 500°C, to thus obtain the trinitrile MTN. The catalyst to be employed is not necessarily limited to the above-mentioned, and other catalysts suited for the ammoxidation, depending upon the conditions, may be usable. As to the reaction temperature and mixed gas composition, moreover, the most preferred may be selected accordingly within the scope of reaction conditions combined therewith.

In order to obtain MTA, it is desirable to rest upon the process of hydrogenating the trinitrile MTN in the presence of a catalyst.

Said hydrogenation is conducted in the liquid phase in the presence of hydrogen, whereby the use of a solvent provides the good results. As the solvent may be usable solely or as a mixture of not less than two kinds, aromatic hydrocarbons such as benzene, toluene and xylene, alcohols such as methanol, ethanol, propanol, isopropanol and isobutanol, ethers such as dioxane and tetrahydrofurane, and other solvents inert under the reaction conditions such as water and liquid ammonia, although the solvents based on alcohols or aromatic hydrocarbon-alcohol mixtures, allowing a reduction in the catalyst amount with a lessened decrease in the yield, are more preferably used. As to the used amount of the solvent, which is not specifically restricted, 50 to 1000 V/W %, preferably 100 to 600 V/W %, relative to the starting MTN provides the satisfactory results. Naturally, the use of larger quantities of the solvent does not interfere with the reaction but, use of a large amount of solvents is not economical from a commercial point of view. By adding a basic substance, for example, a hydroxide or alcoholate of alkali metals such as lithium hydroxide, sodium hydroxide, potassium hydroxide, sodium methylate and sodium ethylate at a rate of 0.05 to 40% by weight, preferably 0.5 to 20% by weight, based on the starting MTN, at the same time, there may be obtained the desirable results such as a decreased addition amount of a catalyst and shortened reaction time. In conducting the hydrogenation, use is made of hydrogen, while a reaction vessel is not specifically limited in design and construction, only if it withstands the reaction conditions, although it is advisable to conduct the reaction in a pressure vessel such as an autoclave in the case of a higher reaction pressure applied. The reaction pressure is in the region of 29,4 to 294 bars G (30 to 300 $Kg/cm^2$ preferably, in the region of 29,4 to 147 bars G (30 to 150 $kg/cm^2G$), whereas the reaction temperature is in the range of −10 to 150°C, preferably, in the range of 40 to 120°C. The hydrogenation is carried out in the presence of a Raney nickel or Raney nickel chromium catalyst.

The novel triamine MTA according to the present invention is colorless crystals at ambient temperature and, upon heating at about 50°C, turns into a colorless, transparent liquid.

In order to obtain the triamine $H_6$MTA of the present invention, either the process of hydrogenating the triamine MTA or direct hydrogenating the trinitrile MTN can be employed.

Hydrogenation of MTA is conducted in the liquid phase in the presence of hydrogen, with an appropriate solvent being employed if necessary. As the solvent may be employed, solely or as a mixture of not less than two kinds, for example, water, ethanol, methanol, propanol, isopropanol, isobutanol, dioxane, acetic acid, or tetrahydrofuran, although water is advantageous in terms of cost and an alcohol-water mixed solvent, in allowing a reduction in the catalyst amount with a lessened decrease in the yield, is preferred. The solvent is not necessarily, among the reaction conditions selected, the requisite one. Yet, when a solvent is used, 0.05 to 10 times the volume, preferably, 0.1 to 5 times the volume, of the triamine MTA may provide the satisfactory results. By adding 0.05 to 20% by weight, preferably, 0.1 to 10% by weight, against MTA of a basic substance, for example, an alkali metal hydroxide such as lithium hydroxide, sodium hydroxide, potassium hydroxide, calcium hydroxide, barium hydroxide and sodium carbonate, or an alkaline earth metal hydroxide or carbonate, there may be obtained the more desirable results. In conducting the hydrogenation, use is made of hydrogen gas, while a reaction vessel is not specifically limited in design and construction, only if it may withstand the corresponding reaction conditions, although it is advisable to conduct the reaction in a pressure vessel such as an autoclave in the case of a higher reaction pressure applied. The reaction pressure is in the region of 4,9 to 294 bars G (5 to 300 $kg/cm^2G$), preferably in the region of 4,9 to 147 bars G (5 to 150 $kg/cm^2G$), whereas the reaction temperature is in the range of −10 to 200°C, preferably, in the range of 50 to 150°C. The hydrogenation is carried out in the presence of a ruthenium catalyst. The catalyst may be supported on a carrier, such as activated carbon, silica gel, alumina, diatomaceous earth and pumice, to obtain a catalyst with the more desirable properties. The ruthenium catalyst allows a reduction in the amount of a catalyst to be added with a lessened decrease in the yield especially when

water containing a small amount of an alkali metal hydroxide or carbonate, alcohol or a mixture thereof is employed as a solvent.

In addition, $H_6$MTA can be directly obtained through hydrogenation of the trinitrile MTN.

In hydrogenating the trinitrile MTN, there may be produced more desirable results by conducting the liquid-phase reaction in the presence of hydrogen and employing a solvent. As examples of the solvent may be mentioned aromatic hydrocarbons such as benzene, toluene and xylene, alcohols such as methanol, ethanol, propanol, isopropanol and isobutanol, ethers such as dioxane and tetrahydrofuran, and acetic acid, liquid ammonia and water. These can be used solely or as a mixture of not less than two kinds, although water, ethanol or their mixture, affording $H_6$MTA in a high yield, is the preferred solvent. When the reaction is conducted in the coexistence of ammonia or in a liquid ammonia solvent, formation of by-products can be prevented, and the similar effect can be achieved by adding to a solvent for the reaction 0.01 to 5% of, preferably, 0.05 to 3.0% of, a basic substance, for example, caustic soda and caustic potash. As to the amount of the solvent to be used, 1 to 10 times the volume of the starting trinitrile MTN, preferably, 1 to 6 times the volume thereof, is the range in which satisfactory results can be produced. In carrying out the hydrogenation, use is made of hydrogen gas, and a reaction vessel is not specifically restricted, only if it may withstand the selected reaction conditions, although it is advisable, in the case of an increased reaction pressure applied, to conduct the reaction in such a pressure vessel as an autoclave. The reaction pressure is in the region of 4,9 to 294 bars G (5 to 300 kg/cm²G), preferably, in the region of 29,4 to 196 bars G (30 to 200 kg/cm²G), while the reaction temperature is in the range of −10 to 250°C, preferably, in the range of 50 to 200°C. The hydrogenation is carried out in the presence of a catalyst. As examples of the catalyst, there may be mentioned a rhodium catalyst. When water, ethanol or a mixture thereof is used as a solvent, especially, there results a higher yield of the hydrogenation reaction, and this may be considered as the most desirable reaction conditions in the case of a one-step production of the triamine $H_6$MTA from the trinitrile MTN.

$H_6$MTA of the present invention is a colorless clear liquid at ambient temperature and, upon cooling, neither solidifies nor produces a precipitate.

The triamines (I) can be used as hardening agents for epoxy resins and corrosion-inhibitors for metals.

The triamines (I) can also be derived into the corresponding triisocyante (II) by a *per se* known procedure of reacting with phosgene.

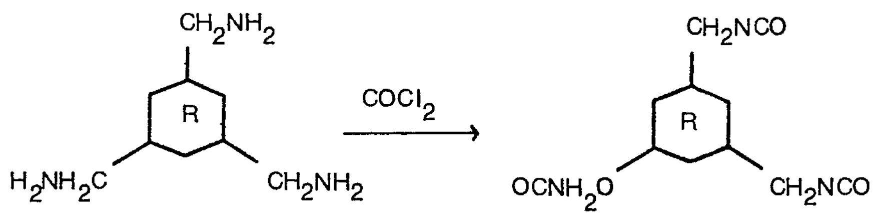


[I] [II]

wherein 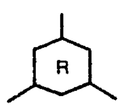 stands for 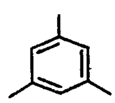 or 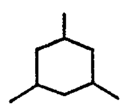

When 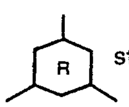 stands for 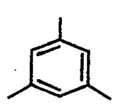

in the above formula [II], the triisocyanate compound is 1,3,5-tris(isocyanatomethyl)benzene (hereinafter referred to as "MTI") and when

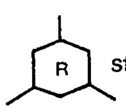 stands for 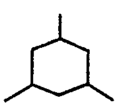

the triisocyanate compound is 1,3,5-tris(isocyanatomethyl)cyclohexane (hereinafter referred to as "$H_6$MTI").

Phosgenation of the triamines represented by the formula (I) can be carried out in accordance with the procedures conventional *per se*. Out of these, one is the procedure termed the so-called cold·hot phosgenation, which comprises adding the triamines [I] or a solution of said triamines in an organic solvent dropwise, under stirring, to cooled liquid phosgene or solution of phosgene in an organic solvent, and raising the reaction temperature with feeding of phosgene to allow the reaction to

proceed and go to conclusion; the other is the process which comprises adding an organic solvent to a salt of the starting triamine to a slurry form or adding an acid to a solution of the triamine in an organic solvent to obtain a slurry of the triamine salt, and elevating gradually the temperature while feeding phosgene to the slurry to allow the phosgenation reaction to proceed and go to conclusion.

The triamines with a high degree of purity may be utilized, although the starting triamines containing a small amount of impurities by-produced during production of said triamines can even be used as well.

As examples of the organic solvent which is useful in the phosgenation reaction may be mentioned aromatic hydrocarbons, halogenated aromatic hydrocarbons, halogenated aliphatic hydrocarbons, halogenated alicyclic hydrocarbons, and, among these, halogenated aromatic hydrocarbons such as chlorobenzene and o-dichlorobenzene are desirable. The salts of the triamines which are operable include acetic acid salt, hydrochloric acid salt, sulfuric acid salt, and carbamic acid salt, and preferred is the carbamic acid salt formed by reacting the triamine with carbon dioxide gas. Phosgene can be used either in the gaseous or liquid form, and phosgene dimer(trichloromethyl chloroformate) which is regarded as a precursor of phosgene in this industrial fields can be used in place of phosgene.

Referring to the reaction temperature for the phosgenation, too much elevated temperature results in formation of a large amount of by-products, while too far reduced temperature leads to a lowered reaction rate, and it is desirable to select the reaction temperature in the range of —20 to 180°C.

Out of the reaction solution with which phosgenation is completed in this manner, excessive phosgene and reaction solvent are removed, and vacuum distillation is then effected, thus resulting in the corresponding triisocyanates (II).

The triisocyanates (II) obtained by the above-mentioned procedure offer various advantages over the conventionally known polyisocyanates. That is to say, they each are an odorless, non-irritant, colorless clear liquid with a very low visocisity at ambient temperature, and are therefore of the great utility as a component for solvent-free or high-solids urethane coatings, while they, being produced from relatively low-priced raw materials in the simplified production process, possess the very high industrial importance.

The triisocyanates (II), through various polyaddition processes utilizing the reaction between an isocyanate and other active hydrogen compound as known in the concerned industrial fields, can produce a wide variety of polyurethane resins. These triisocyanates, though being utilizable directly in the original form, can also be used as various forms of modified products (e.g., dimer, trimer, carbodiimide) and in the form of prepolymers resulting from their reaction with a polyol, poilyamine, aminoalcohol, and water. Where they are directed to the application fields such as backing paint, moreover, they can be utilized in the form of the so-called masked isocyanate formed with a variety of blocking agents being known as well.

In cases where these triisocyanates are reacted with polyol components usually employed in urethane coatings to form coating films, the excellent processability or workability is attained, and the resultant cured coating films exhibit the very good physical properties and weatherability.

Besides being particularly suited for the application field of urethane coatings, furthermore, the above-mentioned triisocyanates derived from the objective compounds of the present invention can be applied in a great variety of isocyanate-based products known in the concerned business circles such as adhesive agents, foamed products, artificial leather and filling agents.

### Reference Example 1

Production of 1,3,5-tricyanobenzene

To 150 parts of a 33% aqueous oxalic acid solution was added 18.2 parts of vanadium pentoxide, followed by heating over a hot water bath at about 100°C, to dissolve vanadium pentoxide. The solution prepared in this manner was referred to as "A solution". Similarly, a solution of 20 parts of chromium oxide (VI) in 150 parts of a 33% aqueous oxalic acid solution was referred to as "B solution". Both A and B solution were mixed homogeneously.

To the mixed solution was added 300 parts of anatasetype titanium dioxide powder baked at 800°C, and water was allowed to vapourize with stirring. The paste thus obtained was molded by wet extrusion of a size of 4 mm diameter and 5 mm length. The resultant moldings were dried at 100°C for 15 hours and then baked in the air at 500°C for 4 hours to prepare a catalyst.

About 200 ml of the catalyst obtained in this manner was filled in a conventional reactor with fixed beds, and a mixed gas consisting of 0.5 mole % of mesitylene, 7 mole % of ammonia and 92.5 mole % of air was reacted under the conditions of the atmospheric pressure and space velocity of 1000 $hr^{-1}$ (converted to the NTP), while maintaining the temperature of a bath for the reactor at 360°C, to give 1,3,5-tricyanobenzene (MTN) in a yield of 51.2 mole %.

### Example 1

In an autoclave of a 300-ml content equipped with an electromagnetic agitator were placed for tight sealing 15 g of 1,3,5-tricyanobenzene (MTN), 15 g of Raney-nickel·chromium catalyst (atomic

ratio of Ni:Cr = 49:1), 27 ml of methanol, 63 ml of m-xylene and 0.18 g of caustic soda, and hydrogen was charged at the initial pressure of 98 bars G (100 kg/cm²G) to conduct the reaction at 100°C, resulting in absorption of 0.59 mole of hydrogen over a 35-minute period. The catalyst was filtrated out and the solvent was distilled off, followed by conducting vacuum distillation, thus resulting in 12.8 g of 1,3,5-tris(aminomethyl)benzene (MTA) as colorless crystals. The substance exhibited the melting point of 49 to 51°C and the boiling point of 136 to 139°C/0,53 bar (0,4 mmHg), with the IR absorption spectrum as illustrated in Fig. 1.

75 mg of MTA obtained by the above-mentioned procedure was dissolved in 100 ml of diethyl ether, and acetic anhydride was added dropwise, whereby there deposited colorless crystals. At the time when no additional crystal formation was observed, addition of acetic anhydride was discontinued, and the crystals were recovered by filtration and dried to give 131 mg of 1,3,5-tris(acetylaminomethyl)benzene. Melting point; 223—225°C.
Elementary analysis (for $C_{15}H_{21}N_3O_3$);

| | C | H | N |
|---|---|---|---|
| Calcd. (%): | 61.84 | 7.27 | 14.42 |
| Found (%): | 61.84 | 7.09 | 14.28 |

400 mg of MTA obtained in the above-mentioned manner was dissolved in 200 ml of diethyl ether, and a diethyl ether solution containing 2.0 g of benzoic anhydride was added, whereupon colorless crystals were separated out immediately. The crystals were recovered by filtration and dried, yielding 1.16 g of crystalline 1,3,5-tris(benzoylaminomethyl)benzene. Melting point; 240—242°C.
Elementary analysis (for $C_{30}H_{27}N_3O_3$);

| | C | H | N |
|---|---|---|---|
| Calcd. (%): | 75.45 | 5.70 | 8.80 |
| Found (%): | 75.16 | 5.77 | 8.69 |

403 mg of 1,3,5-tris(aminomethyl)benzene obtained by the above-mentioned procedure was dissolved in 60 ml of an ethanol/water (3:1) mixed solvent, and carbon dioxide gas was introduced, whereupon colorless crystals separated out. Introduction of carbon dioxide gas was continued until no additional crystal formation was observed, and the crystals were recovered by filtration and dried. Thus, there was obtained 560 mg of colorless crystals with the melting point of 121.5 to 122.5°C.

On the other hand, 330 mg of MTA obtained by the above-mentioned procedure was dissolved in 60 ml of ethanol, and dry hydrochloric acid gas was introduced, whereupon colorless crystals separated out. Introduction of the hydrochloric acid gas was continued until no additional crystal formation was observed, and the crystals were recovered by filtration and dried, resulting in 410 mg of colorless crystals with the melting point of not less than 300°C.

Examples 2 through 10

Procedures were carried out after the manner described in Example 1 under the reaction conditions as shown in Table 1, and the results as exhibited in Table 1 were obtained.

TABLE 1

| Example No. | Catalyst | | Alkali | | Solvent | | $H_2$-press. bars/G ($kg/cm^2$ G) | React. temp. °C | React. time, min. | Yield** mole % |
|---|---|---|---|---|---|---|---|---|---|---|
| | Type | Amt., g | Type | Amt., g | Type | Amt., ml | | | | |
| 2 | R–Ni | 15 | NaOH | 0.25 | MeOH/m-xylene (1/2) | 90 | 98 (100) | 100 | 40 | 76.2 |
| 3 | R–Ni–Cr* | 15 | NaOH | 1.47 | EtOH | 90 | 98 (100) | 72 | 15 | 89.4 |
| 4 | R–Ni | 15 | NaOH | 0.52 | MeOH/toluene (1/1) | 100 | 117,6 (120) | 90 | 30 | 91.0 |
| 5 | R–Ni–Cr* | 7.5 | LiOH. $H_2O$ | 0.37 | EtOH/m-xylene (2/1) | 90 | 107,8 (110) | 100 | 52 | 86.5 |
| 6 | R–Ni–Cr* | 3 | NaOH | 0.4 | MeOH/m-xylene (1/2) | 90 | 117,6 (120) | 105 | 61 | 94.3 |
| 7 | R–Ni–Cr* | 15 | NaOH | 0.27 | i-PrOH | 90 | 117,6 (120) | 100 | 100 | 56.5 |
| 8 | R–Ni–Cr* | 15 | – | – | MeOH/ammonia (1/2) | 138 | 124,5 (127) | 100 | 165 | 41.4 |
| 9 | R–Ni–Cr* | 15 | NaOH | 0.05 | i-ProH/m-xylene (2/1) | 90 | 117,6 (120) | 110 | 180 | 19.1 |
| 10 | 5% Rh–$Al_2O_3$ | 4 | – | – | 25% aqueous ammonia | 80 | 117.6 (120) | 65 | 15 | 89.4 |

Remarks: *): The ratio of Ni:Cr in R–Ni–Cr was 49:1 (atomic ratio).

**): Yields were determined by gas chromatography.

Example 11

Production of 1,3,5-tris(aminomethyl)cyclohexane

In an autoclave of a 300-ml content fitted with an electromagnetic agitator was placed for tight sealing 30 g of 1,3,5-tris(aminomethyl)benzene (MTA), together with 3 g of 5% ruthenium-alumina catalyst (produced by Nippon Engelhard Ltd.), 60 g of water and 0.75 g of caustic soda, and high-pressure hydrogen of 117,6 bars G (120 kg/cm²G) in initial pressure was charged under pressure to react at 115°C for 25 minutes, resulting in absorption of 0.61 mole of hydrogen.

The catalyst was filtrated out, and the solvent was distilled off, followed by vacuum distillation, resulting in 26.8 g of 1,3,5-tris(aminomethyl)cyclohexane ($H_6$MTA). $H_6$MTA was a colorless, clear, less viscous liquid of the boiling point of 127 to 128°C/1,33 mbar (1 mmHg). Its IR spectrum is illustrated in Fig. 2.

In about 20 ml of ethyl ether was dissolved 145 mg of $H_6$MTA obtained in this manner, and acetic anhydride was added dropwise, immediately yielding white crystals as a precipitate. When no additional crystal formation was observed, addition of acetic anhydride was suspended, and the crystals were recovered by filtration and dried to afford 248 mg of a triacetyl derivative. The compound has the melting point of 278 to 279.5°C and was found by elementary analysis to be in agreement with tris(acetylaminomethyl)cyclohexane of $C_{15}H_{27}N_3O_3$, as shown in the following.

Elementary analysis (for $C_{15}H_{27}N_3O_3$);

| | C | H | N |
|---|---|---|---|
| Calcd. (%): | 60.58 | 9.15 | 14.13 |
| Found (%): | 60.66 | 9.22 | 14.03 |

Then, 10 ml of an ethanol solution containing 176 mg of $H_6$MTA was added to a mixture of 20 ml of ethanol and 1 ml of 35% hydrochloric acid, and stirred for about 1 hour. The crystals were recovered by filtration and dried to give 195 mg of a hydrochloride derivative.

The compound had the melting point of not lower than 300°C, and was found by elementary analysis to be in agreement with the trihydrogenchloride of $C_9H_{24}N_3Cl_3$.

Elementary analysis (for $C_9H_{24}N_3Cl_3$);

| | C | H | N | Cl |
|---|---|---|---|---|
| Calcd. (%): | 38.51 | 8.62 | 14.97 | 37.89 |
| Found (%): | 38.39 | 8.83 | 14.73 | 37.94 |

Also, 210 mg of $H_6$MTA was dissolved in 25 ml of ethanol, and carbon dioxide gas was introduced, resulting in separating out of crystals. After carbon dioxide gas was introduced until no additional crystal formation was observed, the crystals were recovered by filtration and dried under reduced pressure at ambient temperature to give 284 mg of white crystals (melting point of 85 to 86.5°C).

Example 12

In an autoclave of 500 ml in inner content fitted with an electromagnetic agitator was placed for tight sealing 100 g of 1,3,5-tris(aminomethyl)benzene together with 5 g of the commercially available 5% ruthenium-carbon catalyst 200 ml of water and 3 g of caustic potash, and the high-pressure hydrogen gas of 117,6 bars G (120 kg/cm²G) was charged under pressure, followed by conducting the reaction at 115°C for 3 hours. 1.82 mole of hydrogen was absorbed, yielding $H_6$MTA in a yield of 84.7 mole %.

Examples 13 through 20

A 20 g of 1,3,5-tris(aminomethyl)benzene was subjected to nuclear reduction in an autoclave of 300 ml in inner content fitted with an electromagnetic agitator under the following reaction conditions, to afford $H_6$MTA in the below-mentioned yield, respectively.

TABLE 1

| Examples | Catalyst | | Solvent*2 | Alkali | | Initial press., bars/G (kg/cm² G) | Reaction temp., °C | Reaction time, min. | Yield of $H_6MTA$, % |
|---|---|---|---|---|---|---|---|---|---|
| | Type*1 | Amount, g | | Type | Amount, g | | | | |
| 13 | A | 1 | Water | $LiOH.H_2O$ | 0.5 | 117,6 (120) | 115 | 23 | 85 |
| 14 | A | 1 | Water | – | – | 117,6 (120) | 115 | 190 | 36 |
| 15 | B | 1 | Water/ethanol (1/2) | NaOH | 0.5 | 117,6 (120) | 115 | 75 | 92 |
| 16 | A | 1 | Water/n-propanol (1/1) | NaOH | 0.5 | 117,6 (120) | 115 | 55 | 87 |
| 17 | B | 1 | Ethanol | NaOH | 0.15 | 117,6 (120) | 135 | 165 | 54 |
| 18 | B | 2 | Isopropanol | NaOH | 0.20 | 117,6 (120) | 145 | 180 | 41 |
| 19 | B | 2 | Ethanol | NaOH | 0.18 | 117,6 (120) | 130 | 128 | 81 |
| 20 | A | 1 | Water | $Na_2CO_3$ | 1.0 | 117,6 (120) | 115 | 36 | 83 |

Remarks:

*1) A; The commercially available 5% ruthenium-alumina catalyst.
B; The commercially available 5% ruthenium-carbon catalyst.

*2) The amount of the solvent is all 80 ml.

Example 21

In an autoclave of a 300-ml content fitted with an electromagnetic agitator was placed for tight sealing 20 g of 1,3,5-tricyanobenzene obtained in Reference Example 1, together with 80 ml of 25% aqueous ammonia, 300 mg of caustic soda and 4 g of a 5% rhodium-alumina catalyst being commercially available, and subjected to reaction under high-pressure hydrogen of 117,6 bars G (120 kg/cm$^2$G) in initial pressure at 105°C for 70 minutes, resulting in absorption of 0.95 mole of hydrogen. By the above procedure was obtained, in a yield of 45%, $H_6$MTA having both the nitrile and benzene ring reduced.

Example 22

In an autoclave of a 300-ml content fitted with an electromagnetic agitator was placed for tight sealing 20 g of 1,3,5-tricyanobenzene obtained in Reference Example 1, together with 40 ml of 25% aqueous ammonia, 40 ml of ethanol, 500 mg of caustic soda and 4 g of a 5% rhodium-alumina catalyst being commercially available, and subjected to reaction under high-pressure hydrogen of 117,6 bars G (120 kg/cm$^2$G) in initial pressure at 105°C for 180 minutes, resulting in absorption of 1.02 moles of hydrogen. By the above procedure was obtained, in a yield of 43%, $H_6$MTA having both the nitrile and nucleus reduced.

Example 23

In an autoclave of a 300-ml content fitted with an electromagnetic agitator was placed for tight sealing 15 g of 1,3,5-tricyanobenzene obtained in Reference Example 1, together 40 ml of ethanol, 40 ml of m-xylene, 320 mg of caustic soda 15 g of Raney nickel-chromium (Ni:Cr = 49:1) and 4 g of a 5% ruthenium-alumina, and subjected to reaction under high-pressure hydrogen of 127,4 bars G (130 kg/cm$^2$G) in initial pressure at 125°C for 150 minutes, to give $H_6$MTA in a yield of 57.2%.

Reference Example 2

Production of 1,3,5-tris(isocyanatomethyl)benzene

In 1200 ml of o-dichlorobenzene in a 2-l four-necked flask was dissolved under warming 90.0 g of 1,3,5-tris(aminomethyl)benzene (MTA). Carbon dioxide gas was introduced in the resultant amine solution until no weight increase was observed, resulting in a slurry of colorless crystals. The slurry material was maintained at a temperature of not higher than 10°C for 30 minutes, while blowing a phosgene gas under stirring, and the temperature was elevated to 130°C over a 2 hour period under feeding of phosgene, followed by maintaining at 130°C for 5 hours. As the phosgenation reaction proceeded, the slurry turned to a solution and, eventually, to a uniform, slightly yellowish, clear solution.

After the completion of the phosgenation reaction, phosgene was released from the solution by blowing nitrogen gas, and the solvent of o-dichlorobenzene was distilled off under reduced pressure. Vacuum distillation of the resultant crude isocyanate gave 112.9 g of 1,3,5-tris(isocyanatomethyl)-benzene (MTI) of the boiling point of 173 to 175°C/0,53 mbars (0,4 mmHg) (the molar yield of 85.2%). The MTI was a less viscous liquid even at 5°C and completely free from odor peculiar to isocyanates. It was found to have the amine equivalent of 83.25 (the theoretical value of 81.1).

Reference Example 3

Production of 1,3,5-tris(isocyanatomethyl)cyclohexane

Phosgenation was carried out in the same manner as in Reference Example 2, except that 70.0 g of 1,3,5-tris(aminomethyl)cyclohexane ($H_6$MTA) (IV) was used in place of 1,3,5-tris(aminomethyl)benzene (MTA) and that the reaction temperature was elevated from 10°C to 120°C over a 6-hour period, followed by maintaining at 120°C for 6 hours. By the above procedure, there was obtained 91.8 g of 1,3,5-tris(isocyanatomethyl)cyclohexane ($H_6$MTI) of the boiling point of 170 to 174°C/0.53 mmHg (molar yield of 90.1%). The $H_6$MTI was a liquid less viscous even at 5°C and free from odor. The found amine equivalent was 84.71 (the calculated one of 83.08).

Experiment Example 1

Using the MTI as obtained in Example 2, a two-can type urethane coating with a highly non-volatile content was prepared.

Component A:

| | |
|---|---|
| 1) Acrylic polyol [a compolymer solution with 65% of nonvolatile content and 65 of OH value produced by copolymerizing in a toluene/butyl acetate mixed solvent (1:1) 50% of styrene, 23.2% of 2-hydroxyethyl methacrylate and 26.8% of n-butyl acrylate] | 863 parts |
| 2) Titanium dioxide powder | 429.5 parts |

3) Ethyl acetate/butyl acetate/cellsolve acetate (1/1/1) — 276.1 parts

Component B:

MTI — 83.3 parts

The component A, as having gotten the pigment well dispersed by means of a ball mill, was mixed with the component B in such a proportion as may realize the NCO/OH= 1/1. The mixture showed 65% of the non-volatile content and 24 seconds of the viscosity, as determined with the use of Ford cup #4 at 25°C. The mixture was immediately spray-applied on a soft steel plate surface treated with phosphoric acid to form a dry coating film of 30 to 40 μ in thickness. After conducting conditioning at 25°C for 7 days, determination of physical properties and weathering test were effected with the coating film.

| | |
|---|---|
| Coating film thickness: | 30 to 40 μ |
| Pencile hardness | H |
| Erichsen extrusion test | 8.5 mm |
| Cross cut test | 100/100 |
| Sunshine type Weather-O-Meter | 500 hr ΔE 1.2 |

Experiment Examples 2 through 4

Using MTI as obtained in Example 2 and $H_6$MTI as obtained in Example 3 in combination with a variety of polyols, two-can type urethane coatings with a high non-volatile content were prepared in the same manner as stated in Experiment Example 1 to investigate physical properties and weatherabilities of resultant coating films. The results obtained were tabulated in Table 2.

TABLE 2

| Experiment No. | 2 | 3 | 4 |
|---|---|---|---|
| Component A: | | | |
| Polyol (parts) | Polyester polyol (I), 255 | Acrylic polyol, 863 | Polyester polyol (II), 243.9 |
| Titanium dioxide (parts) | 225.5 | 430.4 | 219.1 |
| Solvent (parts) | BA, 241.6 | EA/BA/CA (1/1/1), 277.4 | BA, 136.9 |
| Component B (parts) | MTI, 83.3 | $H_6$MTI, 84.7 | $H_6$MTI, 84.7 |
| At the time of mixing of Components, A and B: | | | |
| Non-volatile content (%) | 70 | 65 | 80 |
| Viscosity (sec.) | 24 | 26 | 27 |
| Physical properties: | | | |
| Thickness of coating film ($\mu$) | 30 to 40 | 30 to 40 | 30 to 40 |
| Pencil hardness | HB | H | HB |
| Erichsen (mm) | 8.5 | 8.2 | 8.0 |
| Cross cut test | 100/100 | 100/100 | 100/100 |
| Weatherability, $\Delta E$ | 1.6 | 0.3 | 0.7 |

Remarks:

Polyester polyol (I); A condensate, with 100% of non-volatile content and 22 of OH value, from 2 moles of adipic acid, 1 mole of dipropylene glycol, 2 moles of trimethylol propane and 1 mole of coconut-oil based fatty acid.

Acrylic polyol; A copolymer solution with 65% of non-volatile content and 65 of OH value produced by copolymerizing 50% of styrene, 23.2% of 2-hydroxyethyl methacrylate and 26.8% of n-butyl acrylate in toluene-butyl acetate (1:1).

Polyester polyol (II): A condensate, with 100% of non-volatile content and 230 of OH value, from 2 moles of adipic acid, 1 mole of diethylene glycol, 2 moles of trimethylol propane and 1 mole of coconut-oil based fatty acid.

BA: Butyl acetate

EA: Ethyl acetate

CA: Cellosolve acetate

## Claims

1. A compound of the formula:

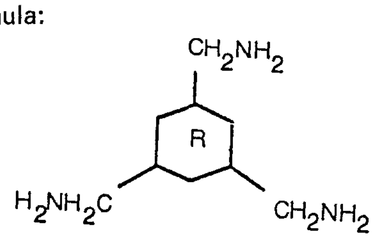

wherein 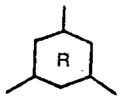 is 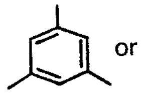 or 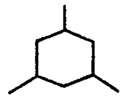

2. A compound according to Claim 1, wherein

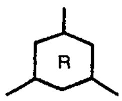 in the formula is 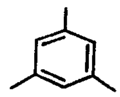

3. A compound according to Claim 1, wherein

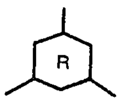 in the formula is 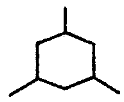

4. A process for production of 1,3,5-tris(aminomethyl)benzene which comprises hydrogenating 1,3,5-tricyanobenzene in the liquid phase in the presence of a Raney-nickel or Raney-nickel-chromium catalyst at 29,4 to 294 bars G and −10 to 150°C.

5. A process according to claim 4, wherein the hydrogenation is carried out in the presence of a basic substance and a solvent.

6. A process for production of 1,3,5-tris(aminomethyl)cyclohexane which comprises hydrogenating 1,3,5-tris(aminomethyl)benzene in the liquid phase in the presence of a ruthenium catalyst at 4,9 to 294 bars G and −10 to 200°C.

7. A process according to claim 6, wherein the hydrogenation is carried out in the presence of a basic substance and a solvent.

**Revendications**

1. Composé de formule:

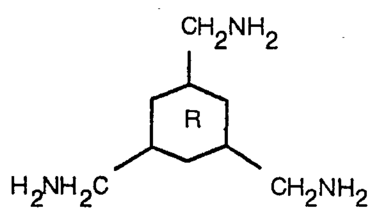

où 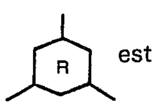 est 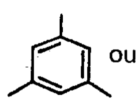 ou 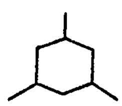

2. Composé selon la revendication 1, dans lequel

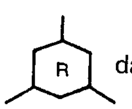 dans la formule est 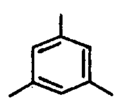

3. Composé selon la revendication 1, dans lequel

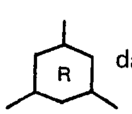 dans la formule est 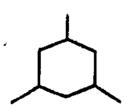

4. Procédé de fabrication du 1,3,5-tris(aminométhyl)benzène, qui consiste à hydrogéner le 1,3,5-tricyanobenzène en phase liquide en présence d'un catalyseur au nickel Raney ou au nickel Raney-chrome entre 29,4 et 294 bars manométriques et entre −10 et 150°C.

5. Procédé selon la revendication 4, dans lequel on effectue l'hydrogénation en présence d'une substance basique et d'un solvant.

6. Procédé de fabrication du 1,3,5-tris(aminométhyl)cyclohexane qui consiste à hydrogéner le

1,3,5-tris(aminométhyl)benzène en phase liquise, en présence d'un catalyseur au ruthénium entre 4,9 et 294 bars manométriques et entre —10 et 200°C.

7. Procédé selon la revendication 6, dans lequel on effectue l'hydrogénation en présence d'une substance basique et d'un solvant.

**Patentansprüche**

1. Verbindung der Formel

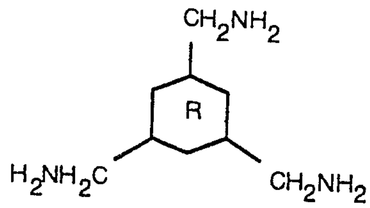

in der 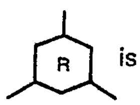 is 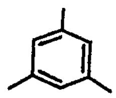 oder 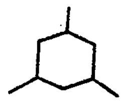 ist.

2. Verbindung nach Anspruch 1, dadurch gekennzeichnet, daß

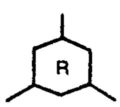 in der Formel 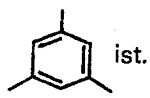 ist.

3. Verbindung nach Anspruch 1, dadurch gekennzeichnet, daß

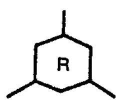 in der Formel 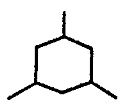 ist.

4. Verfahren zur Herstellung von 1,3,5-Tris(aminomethyl)benzol, dadurch gekennzeichnet, daß 1,3,5-Tricyanobenzol in flüssiger Phase in Gegenwart eines Raney-Nickeloder Raney-Nickel-Chrom-Katalysators bei 29,4 bis 294 bar Manometerdruck und —10°C bis 150°C hydriert wird.

5. Verfahren nach Anspruch 4, dadurch gekennzeichnet, daß die Hydrierung in Gegenwart einer basischen Substanz und eines Lösungsmittels durchgeführt wird.

6. Verfahren zur Herstellung von 1,3,5-Tris(aminomethyl)cyclohexan, dadurch gekennzeichnet, daß 1,3,5-Tris(aminomethyl)benzol in flüssiger Phase in Gegenwart eines Ruthenium-Katalysators bei 4,9 bis 294 bar Manometerdruck und —10°C bis 200°C hydriert wird.

7. Verfahren nach Anspruch 6, dadurch gekennzeichnet, daß die Hydrierung in Gegenwart einer basischen Substanz und eines Lösungsmittels durchgeführt wird.

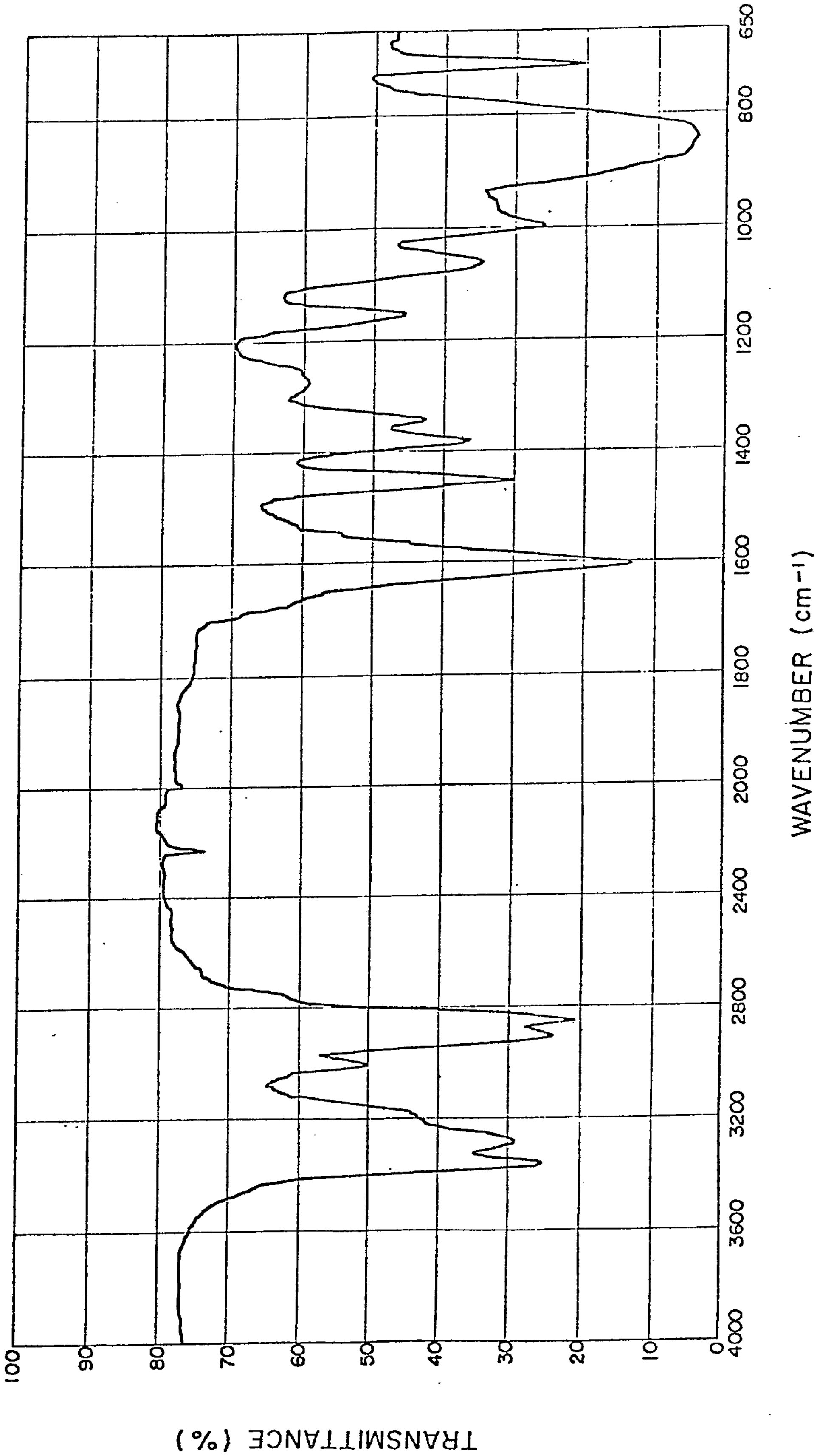

WAVENUMBER (cm-1)
4000
3600
3200
2800
2400
2000
1800
1600
1400
1200
1000
800
650
TRANSMITTANCE (%)
100
90
80
70
60
50
40
30
20
10
0


Fig. 1

Fig. 2

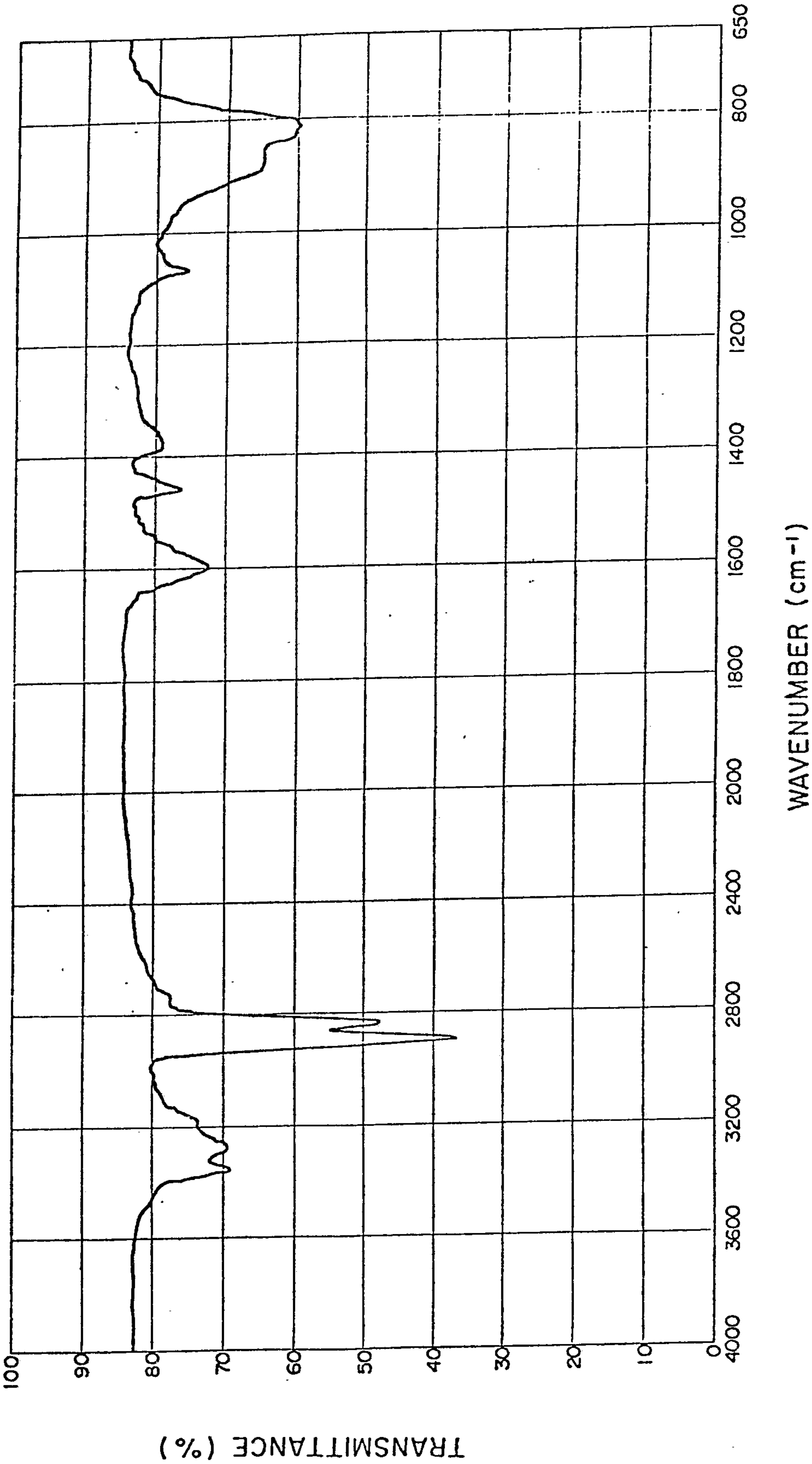
TRANSMITTANCE (%)
100
90
80
70
60
50
40
30
20
10
0
WAVENUMBER (cm⁻¹)
4000
3600
3200
2800
2400
2000
1800
1600
1400
1200
1000
800
650